# FASCICULE DE BREVET EUROPEEN

(11) **EP 1 685 803 B1**
(45) Date de publication et mention de la délivrance du brevet: **14.05.2008**
(21) Numéro de dépôt: 06356007.2
(22) Date de dépôt: 31.01.2006
(51) Int. Cl.: A61B 17/72, A61B 17/74

(54) **Clou huméral**
Humerusnagel
Humerus nail

(30) Priorité: 01.02.2005 FR 0500986
(43) Date de publication de la demande: 02.08.2006
(73) Titulaire: TORNIER, 38330 Saint-Ismier (FR)
(72) Inventeur: Tornier, Alain, 38330 Saint Ismier (FR)
(74) Mandataire: Schouller, Jean-Philippe

(56) Documents cités:
- WO-A-20/04100810
- US-A- 5 653 709
- US-A- 5 658 287
- PATENT ABSTRACTS OF JAPAN vol. 2000, no. 05, 14 septembre 2000 (2000-09-14) & JP 2000 051225 A (YAMADA IKUSHI), 22 février 2000 (2000-02-22)

## Description

La présente invention concerne un clou huméral.

Lors d'une fracture, la partie proximale de l'humérus est susceptible de se briser en plusieurs morceaux. Il est connu de rassembler ces différents fragments au moyen d'un clou huméral, tel que celui visé par l'invention.

Un clou de ce type, par exemple connu de US-A-5,472,444, comprend un corps allongé, destiné à être introduit dans le canal médullaire de l'humérus. Ce corps est creusé de différents perçages, permettant le passage de vis transversales qui assurent la fixation mutuelle du clou et de ce fût huméral.

Ce corps est terminé par une extrémité proximale, dans laquelle sont ménagés au moins trois orifices, décalés angulairement les uns par rapport aux autres. Ces différents orifices reçoivent des vis correspondantes, qui autorisent la solidarisation des différents fragments osseux, initialement éclatés.

Cette solution connue présente cependant certains inconvénients. En effet, les vis de fixation des fragments osseux ont tendance à se dévisser, de sorte que leur position se modifie après implantation, ce qui est susceptible de générer des douleurs chez le patient. Par ailleurs, si ce dévissage survient peu de temps après l'opération chirurgicale, la consolidation de l'ensemble de l'humérus peut se révéler totalement déficiente.

Afin de remédier à cet inconvénient, WO-A-2004/100810 (le préambule de la revendication 1 est basé sur ce document) propose un clou huméral comportant un corps principal, différentes vis de fixation, ainsi qu'un fourreau interne, propre à coulisser dans le volume intérieur du corps. Ce fourreau est par ailleurs creusé de différents orifices, permettant le passage des vis précitées.

En vue de la pose de ce clou, il s'agit tout d'abord de placer le fourreau à l'intérieur du corps de ce clou, puis de faire pénétrer les vis au travers des différents orifices, ménagés respectivement dans le corps et dans le fourreau. Enfin, il s'agit de déplacer le fourreau selon l'axe principal du corps, de façon à bloquer les vis par l'intermédiaire des parois des orifices du fourreau, qui viennent en butée contre les surfaces en regard des vis.

Cette solution alternative présente également des inconvénients. En particulier, la sûreté de la fixation de ce clou dans l'humérus ne se révèle pas toujours satisfaisante.

Ceci étant précisé, l'invention vise à proposer un clou huméral permettant de remédier à ces différents inconvénients.

A cet effet, elle a pour objet un clou huméral, comprenant un corps de forme allongée, propre à être introduit au moins partiellement dans le canal médullaire de l'humérus, ce corps comportant une partie proximale pourvue d'orifices de réception, destinés à recevoir des organes de fixation, tels que des vis, permettant la fixation de fragments huméraux initialement éclatés, ce clou comprenant également un organe de blocage, propre à bloquer lesdits organes de fixation, cet organe de blocage étant mobile par rapport au corps entre une position de libre passage des organes de fixation, dans laquelle cet organe de blocage autorise un libre passage de ces moyens de fixation au travers desdits orifices, et une position de blocage des organes de fixation, dans laquelle cet organe de blocage assure un blocage de ces organes de fixation par rapport aux parois de ces orifices, caractérisé en ce que l'organe de blocage comprend différentes ouvertures, dont chacune comprend une partie de libre passage, s'étendant en regard d'un orifice correspondant dans la position de libre passage, ainsi qu'une partie de blocage, dont les parois sont propres à venir en butée contre un organe de fixation correspondant, dans la position de blocage.

L'invention sera mieux comprise et d'autres avantages de celle-ci apparaîtront plus clairement à la lumière de la description qui va suivre d'un mode de réalisation d'un clou huméral conforme à son principe, donnée uniquement à titre d'exemple non limitatif et faite en se référant aux dessins annexés, dans lesquels :
- la figure 1 est une vue de côté, illustrant un clou huméral conforme à l'invention, une fois implanté ;
- la figure 2 est une vue de côté, illustrant de manière éclatée les différents éléments constitutifs du clou huméral de la figure 1 ;
- la figure 3 est une vue de côté, à plus grande échelle, illustrant côte à côte des orifices et des ouvertures, dont sont respectivement pourvus un corps et un fourreau appartenant au clou huméral des figures précédentes ;
- la figure 4 est une vue en perspective, illustrant une étape intermédiaire de la pose du clou huméral représenté sur les figures précédentes ; et
- les figures 5 et 6 sont des vues de côté, illustrant à grande échelle, selon un angle différent de celui des figures 2 et 3, une partie du corps et du fourreau appartenant au clou huméral des figures précédentes, dans deux positions différentes.

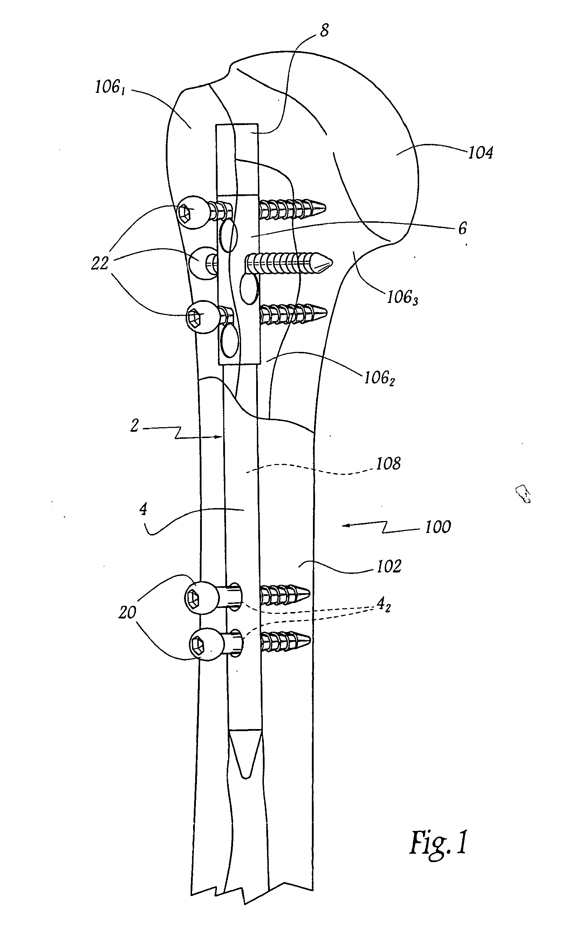

La figure 1 illustre un clou huméral conforme à l'invention, désigné dans son ensemble par la référence 2. Ce clou est destiné, de manière connue en soi, à consolider une fracture intervenue au niveau d'un humérus 100, qui comprend un fût 102 ainsi qu'une tête 104. On note également 106₁ à 106₃ les fragments initialement éclatés de cet humérus, dont le clou 2 conforme à l'invention autorise la consolidation.

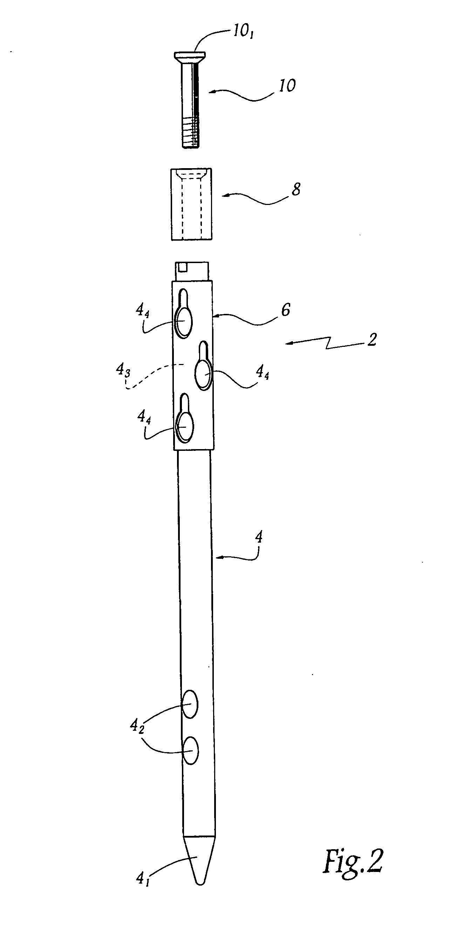

Comme le montre notamment la figure 2, le clou 2 conforme à l'invention comporte tout d'abord un corps allongé 4, affectant une forme de tige droite. A son extrémité distale, ce corps 4, qui est destiné à être introduit dans le canal médullaire 108 de l'humérus, possède une pointe effilée 4₁. De plus, dans sa partie médiane, il est creusé de deux trous traversants 4₂, destinées au passage de vis 20. Ces dernières assurent, de manière connue, la fixation de ce corps 4 par rapport au fût huméral 102.

A l'opposé de la pointe 4₁, ce corps 4 est muni d'une partie proximale 4₃, qui présente une forme cylindrique de section circulaire. Cette partie proximale est creusée de trois orifices traversants 4₄, de forme circulaire, qui sont disposés les uns au-dessous des autres. Ces orifices peuvent avantageusement être décalés angulairement deux à deux, selon l'enseignement de US-A-5,472,444.

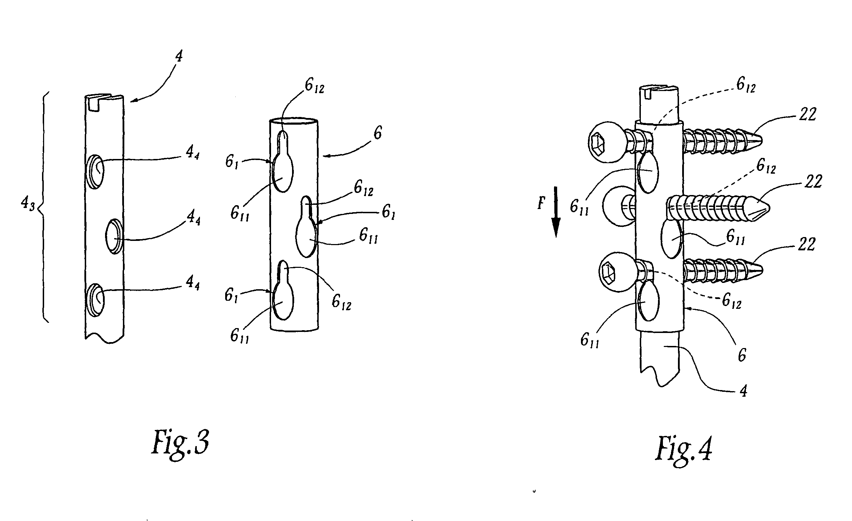

Le clou huméral conforme à l'invention comporte également un fourreau 6, ouvert à ses deux extrémités, qui est plus particulièrement visible sur la figure 3. Ce fourreau 6, qui est de forme cylindrique, présente un diamètre légèrement supérieur à celui de la partie proximale 4₃ du corps 4. Ainsi, ce fourreau 6 est propre à coulisser autour de la surface extérieure de cette partie proximale.

Ce fourreau 6 est creusé de trois ouvertures traversantes 6₁, dont les emplacements correspondent sensiblement à ceux des orifices 4₄. Comme le montre plus précisément la figure 3, qui illustre côte à côte les orifices 4₄ et les ouvertures 6₁, ces dernières possèdent une partie circulaire 6₁₁, dont le diamètre correspond sensiblement à celui des orifices 4₄. Cette partie circulaire 6₁₁ est prolongée par une lumière allongée 6₁₂, dont la section décroît vers l'extrémité fermée, c'est-à-dire supérieure, de cette lumière.

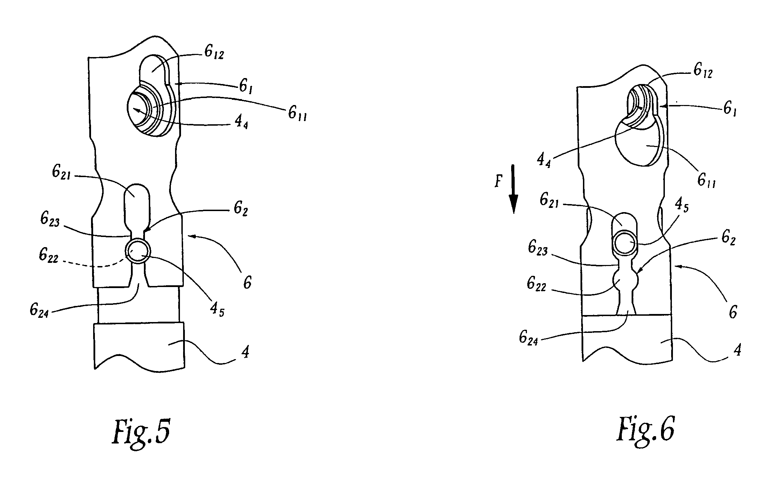

Comme le montrent notamment les figures 5 et 6, le fourreau 6 est également creusé d'une encoche 6₂, qui débouche à son extrémité inférieure sur ces figures, à savoir en direction de la pointe 4₁ du corps 4. Cette encoche définit deux logements 6₂₁ et 6₂₂, respectivement de formes ovale et circulaire, qui sont séparés par un col 6₂₃ de plus faible dimension transversale. Le logement inférieur 6₂₂, de forme circulaire, est prolongé par un canal 6₂₄ débouchant au niveau de l'extrémité inférieure du fourreau 6.

De plus, le corps 4 est pourvu d'un plot 4₅, de section sensiblement circulaire, qui présente des dimensions transversales correspondant globalement à celles des logements 6₂₁ et 6₂₂. Comme on le verra dans ce qui suit, ce plot 4₅ est propre à être reçu, de manière sélective, dans l'un ou l'autre de ces deux logements 6₂₁ et 6₂₂.

Enfin, le clou huméral 2 conforme à l'invention comporte un capuchon creux 8, de forme cylindrique, de même diamètre que celui du fourreau 6. Ce capuchon 8 est propre à être bloqué, comme on le verra dans ce qui suit, au moyen d'une vis 10.

La pose dans l'humérus 100 du clou 2, décrit ci-dessus, va maintenant être expliquée dans ce qui suit.

Il s'agit tout d'abord de rapporter le fourreau 6 autour de la partie proximale 4₃, de manière à faire coïncider les orifices 4₄ avec les parties circulaires 6₁₁ des ouvertures 6₁. On introduit alors l'ensemble formé de ce corps 4 et de ce fourreau 6 dans le canal médullaire 108 de cet humérus 100, de façon connue en soi. A titre de variante, il est possible d'introduire tout d'abord le corps 4 dans le canal médullaire 108, puis de rapporter le fourreau 6 autour de la partie proximale 4₃. De plus, le plot 4₅ du corps 4 est reçu dans le logement inférieur 6₂₂ de l'encoche 6₂, ménagée dans le fourreau 6, ce qui correspond à l'agencement de la figure 5.

Dans ces conditions, il est possible de faire passer, au travers des orifices 4₄, des vis de fixation 22, plus particulièrement visibles sur les figures 1 et 4. Comme cela est connu notamment de US-A-5,472,444, ces vis 22 permettent de solidariser, en vue de leur consolidation, les fragments initialement fracturés 106₁ à 106₃.

Puis on fait coulisser, par exemple manuellement ou grâce à un outil approprié, le fourreau 6 par rapport au corps 4. Dans l'exemple considéré, il s'agit de repousser le fourreau 6 en direction de la partie distale 4₁ du corps 4, grâce au capuchon 8 et à la vis 10.

De façon plus précise, on rapporte le capuchon 8 autour de la partie proximale 4₃ du corps 4, on introduit la vis 10 dans le volume intérieur de ce capuchon 8, puis on fait coopérer cette vis avec un taraudage non représenté, ménagé dans la partie proximale 4₃ du corps 4. Ce vissage de la vis 10 contribue à déplacer le capuchon 8 et, par conséquent, le fourreau 6, selon l'axe principal du corps 4, ce qui est matérialisé par la flèche F sur la figure 4.

On conçoit donc que la tête 10₁ de la vis 10 forme alors une butée pour le capuchon 8, de sorte que celui-ci est immobilisé axialement par rapport au corps 4. Par conséquent, ce capuchon 8 contribue également à bloquer, de manière axiale, le fourreau 6 par rapport à ce corps 4.

Au terme du déplacement évoqué ci-dessus du fourreau 6 par rapport au corps 4, les parties circulaires 6₁₁ ne se trouvent plus en coïncidence avec les orifices 4₄, et les parois des lumières 6₁₂ viennent alors en appui contre les surfaces en regard des vis 22. Les parois de ces lumières 6₁₂, qui forment donc une butée pour les vis 22, empêchent tout mouvement de celles-ci, en particulier celui conduisant à leur dévissage. Les parties circulaires 6₁₁ forment ainsi des parties de libre passage des vis, alors que les lumières 6₁₂ forment des parties de blocage de ces vis.

On notera que les dimensions transversales des parties circulaires 6₁₁ sont supérieures à celles des vis 22, de sorte qu'elles autorisent un libre coulissement de celles-ci. En revanche, les dimensions transversales des lumières 6₁₂ décroissent vers l'extrémité fermée de chaque lumière. Par conséquent, lors du déplacement du fourreau, les parois de ces lumières 6₁₂ viennent coiffer les surfaces en regard des vis 22, de façon à bloquer et à maintenir en position ces dernières.

Par ailleurs, le coulissement du fourreau 6 par rapport au corps 4 induit le déplacement du plot 4₅, depuis le logement inférieur 6₂₂ vers le logement supérieur 6₂₁, ce qui correspond à la position de la figure 6. On notera que ce mouvement, qui s'opère via le col 6₂₃, est autorisé par la nature du matériau constitutif du fourreau 6.

On conçoit que, dans la position de la figure 6, le fourreau 6 se trouve verrouillé par rapport au corps 4. En effet, la présence du col 6₂₃ de faible dimension transversale, empêche le plot 4₅ de retourner vers le logement inférieur 6₂₂, sauf à exercer une force importante sur le fourreau 6. Par conséquent, cette mesure évite tout mouvement intempestif du fourreau 6 par rapport au corps 4. Il est à souligner que, sur cette figure 6, les vis 22 n'ont pas été représentées, dans un but de clarté.

L'invention n'est pas limitée à l'exemple décrit et représenté.

Ainsi, on peut prévoir de remplacer le fourreau 6, s'étendant à l'extérieur du corps 4, par un organe de blocage qui est monté de façon coulissante à l'intérieur de ce corps creux. Cet organe de blocage, de plus petit diamètre, est également pourvu d'ouvertures permettant de bloquer sélectivement les vis de fixation 22.

Par ailleurs, les lumières peuvent s'étendre de manières différentes de celle représentée. Ainsi, elles peuvent prolonger les parties circulaires vers le bas, en biais, ou encore latéralement.

De plus, on peut prévoir d'utiliser plusieurs types de fourreaux, dont les ouvertures présentent des espacements et/ou des orientations différents. Dans cette optique, on peut choisir le fourreau le mieux adapté en fonction de la configuration des fragments osseux qu'il s'agit de rassembler.

Enfin, il est possible de prévoir plus d'une encoche de verrouillage du fourreau 6 par rapport au corps 4, telle que celle 6₂ décrite en référence aux figures 5 et 6.

L'invention permet de réaliser les objectifs précédemment mentionnés.

En effet, la Demanderesse a découvert que la solution décrite dans WO-A-2004/100810 est peu satisfaisante, dans la mesure où elle ne garantit pas un maintien en position fiable des vis de fixation. Ainsi, le blocage de ces vis est assuré par les parois d'orifices circulaires, qui présentent nécessairement une section plus grande que celle des vis, puisque ces dernières doivent coulisser au travers de ces orifices dans la position de libre passage. Dans ces conditions, la force de blocage générée par les parois de ces orifices est relativement peu élevée, de sorte que les vis ont tendance à se déplacer par rapport à leur position initiale.

En revanche, grâce à l'invention, le fourreau 6 assure un blocage fiable des vis de fixation 22, ce qui évite donc tout dévissage intempestif. A cet égard, on notera que la partie de libre passage de chaque ouverture, qui présente des dimensions importantes, autorise un coulissement aisé des vis. De plus, les différentes parties de blocage, de plus faibles dimensions, assurent un maintien en position fiable de ces vis.

## Revendications

1. Clou huméral (2), comprenant un corps (4) de forme allongée, propre à être introduit au moins partiellement dans le canal médullaire (108) de l'humérus (100), ce corps (4) comportant une partie proximale (4₃) pourvue d'orifices de réception (4₄), destinés à recevoir des organes de fixation (22), tels que des vis, permettant la fixation de fragments huméraux (106₁-106₃) initialement éclatés, ce clou comprenant également un organe de blocage (6), propre à bloquer lesdits organes de fixation (22), cet organe de blocage (6) étant mobile par rapport au corps (4) entre une position de libre passage des organes de fixation (22), dans laquelle cet organe de blocage (6) autorise un libre passage de ces moyens de fixation au travers desdits orifices (4₄), et une position de blocage des organes de fixation (22), dans laquelle cet organe de blocage (6) assure un blocage de ces organes de fixation (22) par rapport aux parois de ces orifices (4₄) , **caractérisé en ce que** l'organe de blocage (6) comprend différentes ouvertures (6₁), dont chacune comprend une partie de libre passage (6₁₁), s'étendant en regard d'un orifice (4₄) correspondant dans la position de libre passage, ainsi qu'une partie de blocage (6₁₂), dont les parois sont propres à venir en butée contre un organe de fixation (22) correspondant, dans la position de blocage.

2. Clou huméral selon la revendication 1, **caractérisé en ce que** chaque partie de libre passage (6₁₁) possède des dimensions transversales supérieures à celles des organes de fixation (22), alors que les parties de blocage (6₁₂) présentent des dimensions transversales qui diminuent à l'opposé de chaque partie de libre passage (6₁₁), de façon à coiffer les surfaces en regard des organes de fixation (22) et à les bloquer en position.

3. Clou huméral selon la revendication 2, **caractérisé en ce que** les orifices (4₄) sont circulaires et chaque ouverture (6₁) comprend une partie circulaire de libre passage (6₁₁), sensiblement de même diamètre qu'un orifice correspondant (4₄), ainsi qu'une lumière de blocage (6₁₂) dont la section décroît vers l'extrémité fermée de cette lumière.

4. Clou huméral selon l'une des revendications précédentes, **caractérisé en ce que** la partie proximale (4₃), pourvue desdits orifices de réception (4₄), est cylindrique alors que l'organe de blocage (6) est également cylindrique et présente un diamètre légèrement supérieur ou légèrement inférieur à celui de ladite partie proximale (4₃), de manière à pouvoir coulisser par rapport à cette partie proximale (4₃), à l'extérieur ou à l'intérieur de celle-ci.

5. Clou huméral selon la revendication 4, **caractérisé en ce que** cet organe de blocage (6) est mobile axialement par rapport à la partie proximale (4₃), selon au moins un axe principal de celle-ci, entre lesdites positions de libre passage et de blocage.

6. Clou huméral selon l'une quelconque des revendications précédentes, **caractérisé en ce que** ce clou comporte également des moyens (8) de déplacement de l'organe de blocage (6) vers sa position de blocage, et d'immobilisation de cet organe de blocage dans cette position de blocage.

7. Clou huméral selon l'une des revendications 4 ou 5, prise en combinaison avec la revendication 6, **caractérisé en ce que** les moyens de déplacement et d'immobilisation comprennent un capuchon cylindrique (8), propre à venir en butée contre l'organe de blocage cylindrique (6), dans sa position de blocage, ainsi qu'un organe (10) de maintien en butée de ce capuchon (8) contre cet organe de blocage (6).

8. Clou huméral selon la revendication 7, **caractérisé en ce que** l'organe de maintien en butée est une vis (10), propre à pénétrer dans un volume intérieur du capuchon (8) et à coopérer avec un taraudage du corps (4), une tête (10₁) de cette vis (10) étant propre à maintenir en butée ce capuchon (8) contre l'organe de blocage (6).

9. Clou huméral selon l'une quelconque des revendications précédentes, **caractérisé en ce que** ce clou comporte également des moyens (4₅, 6₂) de verrouillage de l'organe de blocage (6) par rapport au corps (4), dans la position de blocage.

10. Clou huméral selon la revendication 9, **caractérisé en ce que** les moyens de verrouillage de l'organe de blocage (6) par rapport au corps (4) comprennent une encoche (6₂) ménagée dans cet organe de blocage (6), cette encoche (6₂) comportant deux logements (6₂₁, 6₂₂) séparés par un col (6₂₃) de plus faible dimension transversale, ces deux logements étant propres à recevoir, de manière sélective, un plot (4₅) dont est pourvu le corps (4).

## Claims

1. Humeral nail (2) comprising a body (4) of elongated shape adapted to be introduced at least partially into the medullary canal (108) of the humerus (100), this body (4) including a proximal part (4₃) provided with receiving orifices (4₄) intended to receive fixing members (22), such as screws, for fixing initially split humeral fragments (106₁-106₃), this nail also comprising a locking member (6) adapted to lock the said fixing members (22), this locking member (6) being movable relative to the body (4) between a free passage position of the fixing members (22) in which this locking member (6) allows for the free passage of these fixing means through the said orifices (4₄) and a locking position of the fixing members (22) in which this locking member (6) ensures that these fixing members (22) are locked relative to the walls of these orifices (4₄), **characterised in that** the locking member (6) comprises different openings (6₁), each of which comprises a free passage part (6₁₁) extending opposite a corresponding orifice (4₄) in the free passage position, as well as a locking part (6₁₂) the walls of which are adapted to come to bear against a corresponding fixing member (22) in the locking position.

2. Humeral nail according to claim 1, **characterised in that** each free passage part (6₁₁) has greater transverse dimensions than the fixing members (22), while the locking parts (6₁₂) have transverse dimensions which decrease opposite each free passage part (6₁₁) so as to cover the opposing surfaces of the fixing members (22) and lock them in position.

3. Humeral nail according to claim 2, **characterised in that** the orifices (4₄) are circular and each opening (6₁) comprises a circular free passage part (6₁₁) having substantially the same diameter as a corresponding orifice (4₄), as well as a locking port (6₁₂) the section of which decreases towards the closed end of this port.

4. Humeral nail according to one of the preceding claims, **characterised in that** the proximal part (4₃) provided with the said receiving orifices (4₄) is cylindrical, while the locking member (6) is also cylindrical and has a slightly larger or slightly smaller diameter than the said proximal part (4₃) so that it can slide relative to this proximal part (4₃) outside or inside the latter.

5. Humeral nail according to claim 4, **characterised in that** this locking member (6) is movable axially relative to the proximal part (4₃) along at least one main axis of the latter between the said free passage position and the said locking position.

6. Humeral nail according to any one of the preceding claims, **characterised in that** this nail also includes means (8) for displacing the locking member (6) towards its locking position and for securing this locking member in this locking position.

7. Humeral nail according to either of claims 4 or 5 in combination with claim 6, **characterised in that** the displacing and securing means comprise a cylindrical cap (8) adapted to come to bear against the cylindrical locking member (6) in its locking position, as well as a member (10) for holding this cap (8) against this locking member (6).

8. Humeral nail according to claim 7, **characterised in that** the holding member is a screw (10) adapted to penetrate into an inner volume of the cap (8) and to cooperate with a thread of the body (4), a head (10₁) of this screw (10) being adapted to hold this cap (8) against the locking member (6).

9. Humeral nail according to any one of the preceding claims, **characterised in that** this nail also includes means (4₅, 6₂) for locking the locking member (6) relative to the body (4) in the locking position.

10. Humeral nail according to claim 9, **characterised in that** the means for locking the locking member (6) relative to the body (4) comprise a notch (6₂) formed in this locking member (6), this notch (6₂) including two housings (6₂₁, 6₂₂) separated by a neck (6₂₃) having smaller transverse dimensions, these two housings being adapted to receive a stud (4₅) provided on the body (4) as required.

## Patentansprüche

1. Humerusnagel (2), umfassend einen länglich geformten Körper (4), der dafür ausgelegt ist, zumindest teilweise in den Markkanal (108) des Humerus (100) eingeführt zu werden, wobei der Körper (4) einen proximalen Abschnitt (4₃) aufweist, der mit Aufnahme-öffnungen (4₄) versehen ist, die dazu bestimmt sind, Befestigungselemente (22), wie Schrauben, aufzunehmen, die die Befestigung von anfänglich zersplitterten Humerusfragmenten (106₁-106₃) gestatten, wobei der Nagel außerdem ein Blockierungselement (6) umfasst, das dafür ausgelegt ist, die Befestigungselemente (22) zu blockieren, wobei das Blockierungselement (6) in Bezug auf den Körper (4) zwischen einer freien Durchgangs-position der Befestigungselemente (22), in der das Blockierungselement (6) einen freien Durchgang der Befestigungsmittel durch die Öffnungen (4₄) zulässt, und einer Blockierungsposition für die Befestigungselemente (22), in der das Blockierungselement (6) eine Blockie-rung der Befestigungselemente (22) in Bezug auf die Wände der Öffnungen (4₄) sicherstellt, beweglich ist, **dadurch gekennzeichnet, dass** das Blockierungselement (6) verschiedene Öffnungen (6₁) umfasst, von denen jede einen freien Durchgangsabschnitt (6₁₁), der sich gegen-über einer Öffnung (4₄) erstreckt, die der freien Durch-gangsposition entspricht, sowie einen Blockierungs-abschnitt (6₁₂) umfasst, dessen Wände dafür ausgelegt sind, gegen ein entsprechendes Befestigungselement (22) in der Blockierungsposition anzuschlagen.

2. Humerusnagel nach Anspruch 1, **dadurch gekennzeichnet, dass** die freien Durchgangsabschnitte (6₁₁) jeweils transversale Abmessungen besitzen, die größer sind als diejenigen der Befestigungselemente (22), während die Blockierungsabschnitte (6₁₂) transversale Abmessungen aufweisen, welche sich gegenüber den jeweiligen freien Durchgangsabschnitten (6₁₁) verjüngen, so dass die den Befestigungselementen (22) gegenüber-liegenden Oberflächen abgefangen und an Ort und Stelle blockiert werden.

3. Humerusnagel nach Anspruch 2, **dadurch gekennzeichnet, dass** die Öffnungen (4₄) kreisförmig sind und die Öffnungen (6₁) jeweils einen kreisförmigen freien Durchgangsabschnitt (6₁₁) mit im Wesentlichen dem gleichen Durch-messer wie eine entsprechende Öffnung (4₄) sowie ein Blockierungslumen (6₁₂) umfassen, dessen Querschnitt zum geschlossenen Ende des Lumens hin abnimmt.

4. Humerusnagel nach einem der vorhergehenden Ansprüche, **dadurch gekennzeichnet, dass** der proximale Abschnitt (4₃), der mit den Aufnahmeöffnungen (4₄) versehen ist, zylindrisch ist, während das Blockie-rungselement (6) ebenfalls zylindrisch ist und einen etwas größeren oder etwas kleineren Durchmesser als der proximale Abschnitt (4₃) aufweist, so dass es in Bezug auf diesen proximalen Abschnitt (4₃) zu dessen Äußeren oder dessen Inneren hin gleiten kann.

5. Humerusnagel nach Anspruch 4, **dadurch gekennzeichnet, dass** das Blockierungselement (6) in Bezug auf den proximalen Abschnitt (4₃) entlang mindestens einer von dessen Hauptachsen zwischen der freien Durchgangs- und der Blockierungsposition axial beweglich ist.

6. Humerusnagel nach einem der vorhergehenden Ansprüche, **dadurch gekennzeichnet, dass** der Nagel ferner Mittel (8) zum Verschieben des Blockierungselements (6) in seine Blockierungsposition und zur Immobilisierung des Blockierungselements in dieser Blockierungsposition aufweist.

7. Humerusnagel nach einem der Ansprüche 4 oder 5 zusammen mit Anspruch 6, **dadurch gekennzeichnet, dass** die Mittel zum Verschieben und zur Immobilisierung eine zylindrische Kappe (8) umfassen, die dafür ausgelegt ist, gegen das zylindrische Blockierungselement (6) in dessen Blockierungsposition anzuschlagen, sowie ein Element (10), mit dem die Kappe (8) im Anschlag gegen das Blockierungselement (6) gehalten wird.

8. Humerusnagel nach Anspruch 7, **dadurch gekennzeichnet, dass** das Element zum In-Anschlag-Halten eine Schraube (10) ist, die dafür ausgelegt ist, durch ein Innenvolumen der Kappe (8) hindurchzugehen und mit einem Gewinde des Körpers (4) zusammenzuwirken, wobei ein Kopf (10₁) der Schraube (10) dafür ausgelegt ist, die Kappe (8) im Anschlag gegen das Blockierungselement (6) zu halten.

9. Humerusnagel nach einem der vorhergehenden Ansprüche, **dadurch gekennzeichnet, dass** der Nagel außerdem Mittel (4₅, 6₂) zum Sperren des Blockierungselements (6) in Bezug auf den Körper (4) in der Blockierungsposition aufweist.

10. Humerusnagel nach Anspruch 9, **dadurch gekennzeichnet, dass** die Mittel zum Sperren des Blockierungselements (6) in Bezug auf den Körper (4) eine in das Blockierungselement (6) gearbeitete Auskerbung (6₂) umfassen, wobei die Auskerbung (6₂) zwei durch einen Hals (6₂₃) getrennte Auflagen (6₂₁, 6₂₂) mit kleinerer transversaler Abmessung aufweist, wobei diese beiden Auflagen dafür ausgelegt sind, selektiv einen Kontakt (4₅) aufzunehmen, an dem der Körper (4) vorgesehen ist.
